# EUROPÄISCHE PATENTSCHRIFT

(11) **EP 2 699 212 B1**
(45) Veröffentlichungstag und Bekanntmachung des Hinweises auf die Patenterteilung: **12.10.2022**
(21) Anmeldenummer: 12714281.8
(22) Anmeldetag: 30.03.2012
(51) Int. Cl.: A61F 13/66, A61F 13/70, A61F 13/505

(54) **FIXIER- UND TRAGEVORRICHTUNG ZUM SICHEREN AUFSPANNEN EINER WEGWERFBAREN ABSORBIERENDEN INKONTINENZVORLAGE**
FASTENING AND CARRYING DEVICE FOR SECURE MOUNTING OF A DISPOSABLE ABSORBENT INCONTINENCE PAD
DISPOSITIF DE FIXATION ET DE SUPPORT POUR LA TENSION SÛRE D'UNE PROTECTION D'INCONTINENCE ABSORBANTE JETABLE

(30) Priorität: 20.04.2011 DE 102011007819
(43) Veröffentlichungstag der Anmeldung: 26.02.2014
(73) Patentinhaber: Paul Hartmann AG, 89522 Heidenheim (DE)
(72) Erfinder: PAZ, Rui, Miguel, 47800 Krefeld (DE); KESSELMEIER, Ruediger, 89233 Burlafingen (DE); MALOWANIEC, D. Krzysztof, 89522 Heidenheim (DE); DRUMEVA-EBERIUS, Albena, 89129 Langenau (DE); NAGY, Uwe, 89522 Heidenheim (DE); SWEREV, Maximilian, 86169 Augsburg (DE)
(74) Vertreter: DREISS Patentanwälte PartG mbB
(86) Internationale Anmeldenummer: PCT/EP2012/055873
(87) Internationale Veröffentlichungsnummer: WO 2012/143228

(56) Entgegenhaltungen:
- US-A- 4 022 212
- US-A1- 2004 267 225
- US-A1- 2005 131 377
- US-A1- 2005 131 382

## Beschreibung

Die Erfindung betrifft System aus einer Fixier- und Tragevorrichtung mit einer wegwerfbaren absorbierenden Inkontinenzvorlage, gemäß Anspruch 1 mit einem mittels Gürtelverschlusselementen auf sich selbst lösbar schließbaren und so eine in Hüftumfangsrichtung durchgehende Hüftöffnung bildenden Hüftgürtel, an den die Inkontinenzvorlage lösbar fixierbar ist, so dass sie im Schrittbereich des Benutzers getragen und nach Gebrauch wieder vom Hüftgürtel entfernt und verworfen werden kann, wobei der Hüftgürtel einen vorderen Bauchbereich, einen hinteren Rückenbereich und einen linken und einen rechten Seitenbereich umfasst, wobei der Hüftgürtel ausgehend von dem Rückenbereich und dem Bauchbereich je einen vorzugsweise symmetrisch zu einer Längsmittelachse vorgesehenen und sich in einer Längsrichtung in Richtung auf den Schrittbereich des Benutzers erstreckenden Latzabschnitt aufweist, der an seiner körperzugewandten Seite vorzugsweise mechanisch und/oder klebend wirkende Verschlusselemente aufweist, die mit komplementären vorzugsweise mechanisch und/oder klebend wirkenden Verschlusselementen auf der körperabgewandten Seite der Inkontinenzvorlage lösbar haftend zusammenwirken, wobei die Inkontinenzvorlage eine Längsrichtung und eine Querrichtung sowie zwei Längs- und zwei Querkanten aufweist und an ihrer dem Benutzer zugewandten Seite ein Paar in Längsrichtung der Inkontinenzvorlage verlaufende Materialabschnitte aufweist, die entlang ihrer einen Längskante, die als Fixierungslinie dient, an der Inkontinenzvorlage fixiert sind und deren zweite Längskante zumindest abschnittsweise frei ist.

Eine Fixier- und Tragevorrichtung ist beispielsweise bekannt aus WO 2004/069122 A1. Bei dieser bekannten Fixier- und Tragevorrichtung wird der Hüftgürtel im Bauchbereich auf sich selbst geschlossen. In Seitenbereichen links und rechts zwischen Bauchbereich und Rückenbereich erstreckt sich der Hüftgürtel nach unten und bildet dort jeweils einen latzartigen Ansatz, an den eine verhältnismäßig breit ausladende absorbierende Windeleinheit festgelegt werden kann, wobei auf der körperzugewandten Seite der absorbierenden Windeleinheit beidseits vorn und hinten Verschlusselemente hierfür vorgesehen sind, festgelegt werden.

Aus EP 0 700 278 B1 ist eine sogenannte Gürtelwindel mit einem auf sich selbst schließbaren verhältnismäßig schmalen Hüftgürtel bekannt, an dessen körperabgewandter Seite eine nach Art einer Windel anmutende absorbierende Einheit festlegbar ist.

Eine gattungsgemäße Vorrichtung zeigt z.B. die DE 10 2009 049 463 sowie US 2005/0131382 A1.

Der vorliegenden Erfindung liegt die Aufgabe zugrunde, ein System aus einer Fixier- und Tragevorrichtung und einer Inkontinenzvorlage der eingangs genannten Art zu schaffen, die im angelegten Zustand der Inkontinenzvorlage diese sicher aufspannt.

Diese Aufgabe wird ausgehend von einem System mit den Merkmalen des Anspruchs 1 gelöst.

Durch den in Richtung auf den Schrittbereich des Benutzers erstreckten Latzabschnitt bildet der Hüftgürtel zugleich die grundsätzliche Konfiguration eines Höschens, welches zur Vollendung der Höschenform nur noch durch einen Schrittbereich ergänzt zu werden braucht. Dieser Schrittbereich wird bei der erfindungsgemäßen Fixier- und Tragevorrichtung durch die absorbierende Inkontinenzvorlage gebildet, wobei der betreffende hintere und vordere Latzabschnitt mit der körperabgewandten Seite der Inkontinenzvorlage lösbar haftend zusammenwirkt, und zwar unter Verwendung von vorzugsweise mechanisch und/oder klebend wirkenden Verschlusselementen.

Dabei wird erreicht, dass durch die Abstimmung der Breite der Verschlusselemente zum Abstand der Fixierungslinien der Materialabschnitte, die Inkontinenzvorlage im Bereich der Materialabschnitte eine höheren Steifigkeit erhält, die Materialabschnitte sicher beabstandet und die Inkontinenzvorlage damit aufgespannt wird. Auf diese Weise führen die Verschlusselemente der Fixier- und Tragevorrichtung zu einer Versteifung einer Rückenschicht (Backsheet) der Inkontinenzvorlage.

Die Materialabschnitte sind insbesondere solche, die im Inkontinenzbereich als "Cuffs" bezeichnet sind, wobei es sich hierbei um in Längsrichtung verlaufenden bandartige Abschnitte handelt, deren eine Längskante in Form einer sogenannten Cuffsockelfixierung (Fixierungslinien) mit der Inkontinenzvorlage bzw. deren Chassis verbunden ist und wobei die andere Längskante zumindest im Schrittbereich frei ist. Dabei ist insbesondere eine Elastifizierung der freien Längskante des Materialabschnitts vorgesehen, so dass bei bestimmungsgemäßem Gebrauch der Materialabschnitt im Bereich seiner freien Längskante aufgestellt wird und sich so über eine obere körperzugewandte Lage (Topsheet) der Inkontinenzvorlage erhebt. Das heißt, die freie Kante der Materialabschnitte weist im Gebrauchszustand einen Abstand zur oberen körperzugewandten Lage der Inkontinenzvorlage auf. Auf diese Weise kann verhindert werden, dass Flüssigkeit, bevor sie durch die Inkontinenzvorlage aufgesaugt wird, an den Seitenrändern der Vorlage, insbesondere im Bereich der Beinauschnitte, austritt.

Dabei kann es sowohl vorgesehen sein, dass der Abstand der Materialabschnitte hinsichtlich ihrer Fixierungslinien über ihre gesamte Längserstreckung im wesentlichen gleich ist und alternativ kann eine Konturierung vorgesehen sein, so dass insbesondere im Bereich des Schrittes oder auch des Rücken- oder Bauchbereichs die Fixierungslinien der Materialabschnitte einen geringeren Abstand aufweisen als außerhalb dieser Bereiche, so dass sich z.B. im wesentlichen eine sanduhrartige Form ergibt. Durch die Konturierung wird die Anpassung an einen Benutzer noch weiter verbessert. Generell besteht bei bisher auf dem Markt verfügbaren Fixier- und Tragehilfen der Nachteil, das aufgrund z.B. der Bewegung eines Trägers die Gefahr besteht, das sich die gegenüberliegenden Materialabschnitte aufeinander zu bewegen und die flüssigkeitsdurchlässige dem körperzugewandte Lage abdecken, wodurch die Produktleistung beeinträchtigt wird.

Durch die Erfindung wird die Inkontinenzvorlage aufgespannt, so dass die gesamte Fläche zwischen den Fixierungslinien der Materialabschnitte zur Aufnahme von Flüssigkeit zur Verfügung steht, um die ganze Produktkapazität hinsichtlich der Aufnahme und Absorptionsfähigkeit ausnutzen zu können. Ein weiterer Vorteil besteht darin, dass das Verschlusselement einen gleichmäßigen Zug auf den Schrittbereich der Inkontinenzvorlage ausübt und dieser damit über die gesamte Fläche zwischen den Fixierungslinien der Materialabschnitte gleichmäßig in Richtung des Schrittbereiches des Benutzers gezogen wird und somit ein zu enges Hineinziehen der Inkontinenzvorlage in den Schrittbereich des Benutzers in der Mehrzahl der Tragesituationen verhindert werden kann.

Sofern die Fixierungslinien nicht parallel zueinander verlaufen, bezieht sich die Angabe der Prozent auf den Bereich des geringsten Abstandes der Fixierungslinien im Schrittbereich. Unter dem Schrittbereich der Inkontinenzvorlage soll der Bereich verstanden werden, der sich ausgehend von der Mitte der Vorlage, gesehen in Längsrichtung, jeweils 25% in Richtung der vorderen und hinteren Teils der Vorlage erstreckt, das heißt, einen Bereich von 50% der Vorlage um deren Längsmitte umfasst.

Insbesondere kann vorgesehen sein, dass die Breite der Verschlusselemente quer zur Längsrichtung mindestens 80% des Abstands der Fixierungslinien der Materialabschnitte der Inkontinenzvorlage beträgt.

Bei einer Konturierung der Fixierungslinien der Materialabschnitte kann vorgesehen sein, dass der größte Abstand der Fixierungslinien im Rückenbereich der Inkontinenzvorlage beispielsweise bei 180 mm bis 200 mm, vorzugsweise bei 192 mm liegt, während der kleinste Abstand im Schrittbereich bei 135 mm bis 145 mm, vorzugsweise bei 140 mm liegt. Ist bei einer Konturierung der Fixierungslinien der Abstand der Fixierungslinien im hinteren oder vorderen Teil im Bereich einer Querkante der Vorlage geringer, so ist auch dann der geringste Abstand der Fixierungslinien im Schrittbereich maßgeblich. Der Abstand der Materialabschnitte im hinteren oder vorderen Teil im Bereich einer Querkante der Vorlage kann beispielsweise bei 75 mm bis 85 mm, vorzugsweise bei 81 mm liegen. Diese Angabe beziehen sich auf die Fixierungslinien in Bereichen, in denen die zweite Längskante der Materialabschnitte frei ist. Darüber hinaus kann die zweite Längskante der Materialabschnitte neben den freien Bereichen auch Bereiche umfassen, in denen sie an der Vorlage festgelegt ist.

Diesem Wunsch nach Versteifung der Rückenschicht (Backsheet), die dem Benutzer abgewandt ist, steht nun entgegen, dass eine zu weit gehende Versteifung bis in die Randbereiche der Vorlage hinein zu einem Kontakt der Verschlusselemente mit der Haut des Trägers führen könnte und insgesamt bei einer Bewegung als unangenehm empfunden wird. Es ist daher gewünscht, dass an den Rändern der Inkontinenzvorlage keine Versteifung auftritt, so dass bei Bewegung der Beine die Bereiche der Inkontinenzvorlage, die sich rechts und links vom angebrachten Verschlusselement erstrecken, die Haut vor dem Kontakt mit den insbesondere mechanischen Verschlusselementen schützen, wobei auch bei Adhäsivverschlusselementen ein Kontakt nicht erwünscht ist, da es hier zu einem Verkleben mit der Haut kommen kann, das vom Benutzer ebenfalls als unangenehm empfunden wird. Insbesondere bildet sich rechts und links des Verschlusselementes eine Art Wulst, die den Kontakt des Verschlusselements mit dem Benutzer verhindert. Es ist daher vorgesehen, dass die Breite der Verschlusselemente quer zur Längsrichtung 60 bis 90%, weiter bevorzugt 70 bis 80%, weiter bevorzugt 74 bis 76 % des Abstands der Fixierungslinien der Materialabschnitte der Inkontinenzvorlage beträgt.

Die Versteifung der Rückenschicht (Backsheet) und damit der gesamten Inkontinenzvorlage im Bereich, der auf der Rückenschicht fixierten Verschlusselemente der Latzabschnitte der Fixier- und Tragevorrichtung wird gemessen als Kraft, die aufgebracht werden muss, um einen entsprechenden Probenkörper gegen seine Rückstellkraft und Eigenstabilität zu verbiegen (Biegesteifigkeit). Die Fixier- und Tragevorrichtung erhöht die sogenannte Biegesteifigkeit der Inkontinenzvorlage um mindestens den Faktor 2, vorzugsweise mindestens den Faktor 4, weiter bevorzugt mindestens den Faktor 6 und vorzugsweise höchstens um den Faktor 18. Die Biegesteifigkeit einer Vorlage von beispielsweise 400 mN wird durch Anbringen der Fixier- und Tragevorrichtung auf mindestens 800 mN, vorzugsweise mindestens 1.600 mN, weiter bevorzugt mindestens 2.400 mN und vorzugsweise auf höchstens 7.200 mN erhöht.

Des weiteren kann besonderes bevorzugt vorgesehen sein, dass die Breite der Verschlusselemente quer zur Längsrichtung mindestens 30% und höchstens 90%, vorzugsweise mindestens 50% und höchstens 80% der Breite der Inkontinenzvorlage in dem Bereich, in dem die Verschlusselemente mit der Inkontinenzvorlage zusammenwirken, aufweisen. Hierdurch wird, wie bereits angegeben, erreicht, dass es nicht zu einem Scheuern der Verschlusselemente im Beinbereich kommt, sondern auch bei einer Bewegung des Trägers der Beinbereich geschützt ist. Die Breite der Inkontinenzvorlage an der breitesten Stelle des Produktes beträgt von 200 mm bis 400 mm, vorzugsweise 220 mm bis 320 mm, die Breite des absorbierenden Kerns beträgt von 180 mm bis 380 mm, vorzugsweise 200 mm bis 300 mm. So beträgt beispielsweise die Breite der Inkontinenzvorlage MoliForm^{®} Premium Soft Light (Größe 1) 260 mm, die Breite des absorbierenden Kerns 242 mm, bei MoliForm^{®} Premium Soft Extra (Größe 3) beträgt die Breite der Vorlage 300 mm, die Breite des absorbierenden Kerns 260 mm.

Der Hüftgürtel umfasst in Hüftumfangsrichtung einen Bauchbereich sowie einen Rückenbereich und zwei Seitenbereiche, die jeweils eine Erstreckung in Hüftumfangsrichtung aufweisen und sich in Hüftumfangsrichtung aneinander anschließen bei Bildung eines geschlossenen Hüftgürtels.

In Längsrichtung besitzen Bauch- und Rückenbereich sowie die Seitenbereiche eine proximale und eine distale Querkante, die gerade oder gekrümmt, insbesondere auch nur bereichsweise gekrümmt ausgebildet sein können, zur Ausbildung einer Konturierung.

Schließlich wird die distale Erstreckung des Bauch- und Rückenbereichs und damit die distale Querkante des Bauch- und Rückenbereichs definiert durch eine gedachte Linie in Querrichtung am proximalsten Punkt der distalen Begrenzung des Hüftgürtels, beispielsweise eine Tangente. Der proximalste Punkt kann dabei in Hüftumfangsrichtung im Bauch-, Rücken- oder Seitenbereich liegen. Dabei ist die Fläche proximal dieser distalen Querkante ein Teil des Bauch- bzw. Rückenbereichs und die Fläche distal dieser distalen Querkante ein Teil des jeweiligen Latzabschnitts.

Als Längsrichtung des Hüftgürtels wird dabei die Längsrichtung an einem stehenden Benutzer definiert, die Querrichtung ist dabei senkrecht hierzu und fällt mit der Hüftumfangsrichtung zusammen. Die Längsrichtung der Inkontinenzvorlage entspricht der Richtung deren Längserstreckung, wobei die Querrichtung hierzu senkrecht verläuft.

Besonders bevorzugt weist die Inkontinenzvorlage zwei Längs- und zwei Querkanten auf, wobei im an der Fixier- und Tragevorrichtung festgelegten Zustand die Querkanten der Inkontinenzvorlage vorzugsweise im Wesentlichen einer Querkante, insbesondere der dem Schrittbereich abgewandten, also proximalen Querkante des Hüftgürtels entsprechen. Das heißt, die proximale Kante des Bauch- oder Rückenbereichs fällt mit einer Querkante der Inkontinenzvorlage in angelegten Zustand vorzugsweise zusammen. Im Falle einer konturierten proximalen Querkante des Hüftgürtels, beispielsweise durch einen Bauchausschnitt, läuft die proximale Kante parallel zur Hüftumfangsrichtung durch den distalsten Punkt dieser Konturierung. Auf diese Weise kann einfach eine Markierung bereitgestellt werden, anhand derer die optimale Anlegung des Hüftgürtels sowie der Inkontinenzvorlage am Hüftgürtel erreicht werden kann und somit auch für die jeweilige Größe optimale Positionierung der Verschlusselemente, um einen auf der einen Seite optimalen Aufspanneffekt der Materialabschnitte und damit der dem Träger zugewandten Lage zu erreichen und auf der anderen Seite einen Kontakt der Verschlusselemente mit dem Benutzer zu verhindern. Vorzugsweise sind die Verschlusselemente in dieser Position 100 mm bis 180 mm, vorzugsweise 120 mm bis 170 mm, weiter bevorzugt 140 mm bis 160 mm im Bereich des vorderen Latzabschnitts und vorzugsweise 170 mm bis 250 mm, vorzugsweise 190 mm bis 240 mm, weiter bevorzugt 210 mm bis 230 mm im Bereich des hinteren Latzabschnitts von der proximalen Querkante des Hüftgürtels entfernt.

Zur Verbesserung der Passform und für eine einfache Handhabung erweist es sich als vorteilhaft, dass der betreffende Latzabschnitt sich in Richtung auf den Schrittbereich verjüngt.

Weiter erweist es sich als vorteilhaft, wenn die Verschlusselemente des Latzabschnitts an einem schrittzugewandten Ende des Latzabschnitts vorgesehen sind. Darüber hinaus können am hinteren und/oder vorderen Latzabschnitt ein oder mehrere weitere Verschlusselemente vorgesehen sein, die zwischen dem schrittzugewandten Ende und dem Bauch- bzw. Rückenbereich positioniert sind und die der zusätzlichen Fixierung der Inkontinenzvorlage dienen.

Weiter erweist es sich als vorteilhaft, wenn die vorzugsweise mechanisch und/oder klebend wirkenden Verschlusselemente des Latzabschnitts, insbesondere am schrittzugewandten Ende des Latzabschnitts, auf einem streifenförmigen und quer zur Längsrichtung erstreckten Materialabschnitt vorgesehen sind, der an den Latzabschnitt angefügt ist. Auf diese Weise kann der Latzabschnitt über seine gesamte flächenhafte Erstreckung in Querrichtung in einer bestimmungsgemäßen zum Benutzer symmetrischen Weise an der Außenseite, auch Rückenschicht oder Backsheet, der Inkontinenzvorlage lösbar festgelegt werden. Der Latzabschnitt kann so weitgehend faltenfrei und wie erwähnt zu den Beinen und zum Schrittbereich bzw. zu einer auf den Benutzer abstellenden Längsmittelachse korrekt ausgebreitet und fixiert werden.

Vorzugsweise haben die mechanisch und/oder klebend wirkenden Verschlusselemente des Latzabschnitts eine Erstreckung in Querrichtung von 8 cm bis 14 cm, vorzugsweise 10 cm bis 12 cm, vorzugsweise 11 cm und vorzugsweise eine Erstreckung in Längsrichtung von 0,5 cm bis 5,0 cm, vorzugsweise 1,0 cm bis 4,0 cm, vorzugsweise 1,0 cm bis 3,0 cm, vorzugsweise 1,5 cm. Damit ist eine ausreichend große Fläche für die Haftverbindung der mechanisch und/oder klebend wirkenden Verschlusselemente des Latzabschnitts mit der Außenseite der Inkontinenzvorlage für einen sicheren Produktsitz gewährleistet.

Die Außenseite der Inkontinenzvorlage ist dabei so auszubilden, dass sie mit den Verschlusselementen des Latzabschnittes korrespondiert, das heißt, bei einem mechanischen Verschlusselement in Form von Haken wird die Außenseite der Inkontinenzvorlage vorzugsweise durch eine schlaufenbildende Komponente, vorzugsweise durch ein Vlies, insbesondere die Vlieslage eines Vlies-Folien-Laminates, gebildet, während bei einem klebenden Verschlusselement die Außenseite der Inkontinenzvorlage vorzugsweise durch eine Folienlage gebildet wird, auf der die klebenden Verschlusselemente anhaften können.

Nach einem weiteren Erfindungsgedanken erweist es sich als vorteilhaft, wenn die Verschlusselemente des Latzabschnitts, insbesondere am schrittzugewandten Ende, vor der Ingebrauchnahme auf die körperzugewandte Seite des Latzabschnitts eingeschlagen und in dieser Konfiguration leicht lösbar an der körperzugewandten Seite des Latzabschnitts haftend sind. Auf diese Weise sind die an dem Latzabschnitt vorgesehenen, insbesondere mechanisch und/oder klebend wirkenden Verschlusselemente während des Anlegens des Hüftgürtels, insbesondere bei pflegebedürftigen Personen, so lange deaktiviert, wie sie nicht zum Festlegen der Inkontinenzvorlage benötigt werden. Sie behindern dabei nicht das Anlegen des Hüftgürtels, indem sie am Hüftgürtel oder an anderen Komponenten wie Bettlaken, Patientenunterlagen oder dergleichen, festhaken. In der Praxis kann es sich nämlich als vorteilhaft erweisen, wenn der vordere und/oder hintere Latzabschnitt auf sich selbst und/oder auf den Bauch- oder Rückenbereich gefaltet ist und die Faltung erst nach Anlegen des Hüftgürtels und der Inkontinenzvorlage geöffnet wird, um die Vorlage zu fixieren.

Zusätzlich kann diese Faltung der Latzabschnitte durch Fixierpunkte festgelegt werden. Dies kann zum Beispiel mittels einer Ultraschallverbindung, insbesondere kleiner separater Schweißpunkte erfolgen, die im Randbereich der überlappenden Lagen befestigt werden, aber auch mittels Klebeverbindungen, insbesondere Klebepunkten. Andere Arten der Anhaftung sind ebenfalls umfasst. Die Öffnungskraft dieser zusätzlichen Fixierung der Latzfaltung liegt signifikant unterhalb der Materialfestigkeit des Hüftgürtels und zerstört beim Auffalten der Latzabschnitte nicht das Material des Hüftgürtels.

Im Hinblick auf die praktische Handhabbarkeit erweist es sich auch als vorteilhaft, wenn der Hüftgürtel nur in einem Bereich öffenbar und schließbar und die Gürtelverschlusselemente in diesem Bereich an freien Endabschnitten des Hüftgürtels vorgesehen sind. Der Hüftgürtel ist also langgestreckt und hat zwei Endabschnitte, die mittels der Gürtelverschlusselemente aufeinander schließbar sind, um den Hüftgürtel um den Körperumfang des Benutzers herum anlegen zu können.

In Weiterbildung dieses Erfindungsgedankens erweist es sich als vorteilhaft, wenn im anderen Seitenbereich, in dem der Gürtel also nicht öffenbar und schließbar ist, Sekundärverschlusselemente vorgesehen sind, mittels derer eine Umfangslänge des Hüftgürtels einstellbar und so zumindest der vordere Latzabschnitt symmetrisch zum Schritt des Benutzers positionierbar ist. Vorzugsweise sind die Sekundärverschlusselemente so ausgebildet, dass sie im angelegten Zustand der Fixier- und Tragevorrichtung in der Ansicht optisch und/oder haptisch nicht von den Gürtelverschlusselementen unterscheidbar sind. Es kann sich jedoch auch als vorteilhaft erweisen, wenn die Sekundärverschlusselemente von den Gürtelverschlusselementen optisch und/oder haptisch unterscheidbar sind, um auf die unterschiedliche Funktion der Verschlusselemente hinzuweisen. Dies kann beispielsweise durch unterschiedliche Farben, Formen, Textur oder auf andere Weise erfolgen. Die Gürtelverschlusselemente und die Sekundärverschlusselemente sind dabei vorteilhafter Weise beidseits des Bauchbereichs und/oder in Seitenbereichen des Hüftgürtels vorgesehen.

Beim Anlegen des Hüftgürtels führt die Pflegeperson den geöffneten langgestreckten Hüftgürtel bei einem liegenden Patienten unter dessen Körper auf Hüft- oder Rückenhöhe hindurch. Sodann wird der Gürtel zur Bildung der geschlossenen Hüftöffnung mittels der Gürtelverschlusselemente auf sich selbst geschlossen. In diesem Zustand ist der Gürtel zwar angelegt, jedoch noch nicht durch Aktivierung der Sekundärverschlusselemente in seine optimale Passform gebracht. Beispielsweise wird in diesem Zustand der bauchseitige Latzabschnitt durch den Zug in Hüftumfangsrichtung, der durch das Schließen der Gürtelverschlusselemente ausgeübt wird, zu der betreffenden Seite hin gezogen. Dem kann nun durch Einstellen der optimalen Hüftumfangslänge und einer geeigneten Spannung in Hüftumfangsrichtung begegnet werden, indem die Sekundärverschlusselemente entsprechend optimal eingestellt werden. Mittels dieser Sekundärverschlusselemente werden also - wie bereits ausgeführt - nicht zwei offene freie Enden aufeinander geschlossen, sondern es wird die Umfangslänge des Hüftgürtels gegebenenfalls verstellt.

Die Sekundärverschlusselemente können in verschiedener Weise verwirklicht sein. Nach einer bevorzugten Ausführungsform der Erfindung wird vorgeschlagen, dass eine Komponente der Sekundärverschlusselemente auf einem an den Hüftgürtel angefügten Materialabschnitt vorgesehen ist und die andere Komponente an einer Außenseite des Hüftgürtels selbst vorgesehen ist. Solchenfalls wird also der Hüftgürtel als solcher nicht verändert, sondern er erhält ein zusätzliches Element in Form des die Sekundärverschlusselemente tragenden Materialabschnitts, der in an sich beliebiger Weise auf das Material des Hüftgürtels aufgebracht werden kann, beispielsweise durch Aufnähen, Aufkleben, Aufsiegeln, Ultraschallschweißen oder dergleichen übliche Fügeverfahren.

Es erweist sich auch als vorteilhaft, wenn sich die Gürtelverschlusselemente und/oder die Sekundärverschlusselemente über im Wesentlichen die gesamte Erstreckung des Hüftgürtels in Längsrichtung, also über die gesamte Breite des Hüftgürtels, erstrecken. Auf diese Weise kann beim Einstellen der Sekundärverschlusselemente ein gleichmäßiger Zug über die gesamte Breite des Gürtels auf den Gürtel ausgeübt werden.

Es ist auch denkbar, dass sich die Gürtelverschlusselemente und/oder die Sekundärverschlusselemente zu ihrem freien Ende hin verjüngen oder verschmälern, um ein Reiben der Ecken dieser Verschlusselemente auf der Haut des Benutzers zu minimieren.

In einer alternativen Ausführungsform ist es darüber hinaus denkbar, dass die Gürtelverschlusselemente und/oder die Sekundärverschlusselemente sich nicht über im Wesentlichen die gesamte Breite des Hüftgürtels erstrecken. In einem solchen Fall ist es besonders vorteilhaft, wenn die Quererstreckung der Gürtelverschlusselemente und/oder der Sekundärverschlusselemente größer ist als ihre Längserstreckung.

Vorzugsweise stehen die Gürtelverschlusselemente und/oder die Sekundärverschlusselemente über den Seitenrand des Hüftgürtels um mindestens 30 mm, insbesondere um mindestens 40 mm und weiter insbesondere um mindestens 50 mm in Querrichtung über. Die Erstreckung der Gürtelverschlusselemente und/oder die Sekundärverschlusselemente in Längsrichtung der Fixier- und Tragevorrichtung beträgt insbesondere mindestens 25 mm, weiter insbesondere mindestens 35 mm und weiter insbesondere mindestens 45 mm. Damit ergibt sich ein bevorzugtes Verhältnis von Quererstreckung zu Längserstreckung von 30 zu 25, vorzugsweise von 40 zu 35 und insbesondere von 50 zu 45. Insbesondere kann vorgesehen sein, dass die Gürtelverschlusselemente und/oder die Sekundärverschlusselemente trapezförmig ausgebildet sind, insbesondere mit einem Seitenverhältnis von 50mm in Querrichtung zu 35/30mm in Längsrichtung, wobei die kürzere Seite des Trapezes die freie Seite der Verschlusselemente bildet. Damit ist eine ausreichend große Fläche für die Haftverbindung der Gürtelverschlusselemente und/oder der Sekundärverschlusselemente mit der Außenseite des Hüftgürtels für einen sicheren Produktsitz gewährleistet.

Dabei können die Gürtelverschlusselemente und/oder die Sekundärverschlusselemente sogenannte Anfass- oder Griffabschnitte umfassen oder mit diesen verbunden sein. Die Anfass- oder Griffabschnitte sind vorzugsweise am freien Ende der Gürtel- oder Sekundärverschlusselemente vorgesehen und weisen keine Verschlussmittel z.B. in Form von mechanischen und/oder klebenden Elementen auf. Hierdurch wird die Anfassbarkeit der Verschlussmittel verbessert.

In Weiterbildung der Erfindung wird vorgeschlagen, die Gürtelverschlusselemente und/oder die Sekundärverschlusselemente zumindest abschnittsweise elastisch auszubilden.

Zum Verkürzen der Umfangslänge des Gürtels unter Verwendung der Sekundärverschlusselemente wird dabei vorzugsweise eine Z-förmige Faltung des Gürtels durch Schließen der Sekundärverschlusselemente bewirkt, die durch den dabei entstehenden Druck des Hüftgürtels gegen die Körperoberfläche des Benutzers und/oder durch Reibung der aneinander anliegenden Abschnitte der Z-förmigen Faltung gehalten wird.

Im Hinblick auf die Schaffung einer guten Passform und eines hohen Tragkomforts erweist es sich als vorteilhaft, wenn der Hüftgürtel mindestens einen elastischen Abschnitt, vorzugsweise in jedem Seitenbereich einen elastischen Abschnitt umfasst, so dass der Hüftgürtel in Hüftumfangsrichtung elastisch dehnbar ist.

Es erweist sich weiter als vorteilhaft, wenn der Hüftgürtel im Bauchbereich und/oder im Rückenbereich im Wesentlichen undehnbar ausgebildet ist.

In einer alternativen Ausführungsform kann es sich jedoch auch als vorteilhaft erweisen, wenn sich ein oder mehrere elastische Abschnitte im Bauchbereich und/oder Rückenbereich befinden, vorzugsweise in Form einer keilförmigen, trapezförmigen oder viereckigen Elastifizierung.

Eine besonders bevorzugte alternative Ausführungsform weist zwei trapezförmige Elastifizierungen im Rückenbereich auf, die insbesondere als elastisch dehnbare Materialabschnitte ausgebildet sind, die jeweils in den Bereichen des Rückenbereichs vorgesehen sind, die an die Seitenbereiche angrenzen und zwischen sich einen nicht elastischen Bereich einschließen. Dabei können die beiden parallelen Kanten der elastischen Materialabschnitte in Längsrichtung angeordnet sein und die beiden weiteren Kanten mit einer proximalen Querkante des Hüftgürtels und mit einer auf den Schrittbereich gerichteten Kante des Rückenbereichs zusammenfallen. Je nach Breite der elastischen Bereich kann vorgesehen sein, dass diese sich in Längsrichtung bis in den Latzabschnitt, insbesondere den hinteren Latzabschnitt erstrecken.

Im Hinblick auf die Bemessung des Hüftgürtels erweist es sich als vorteilhaft, wenn der Hüftgürtel an vorzugsweise jeder Stelle eine Erstreckung in Längsrichtung von wenigstens 5 cm, insbesondere von wenigstens 6 cm, insbesondere von wenigstens 7 cm, insbesondere von wenigstens 8 cm aufweist. Hierbei ist die Abmessung des Hüftgürtels in Längsrichtung, also die Gürtelbreite quer zu seiner Umfangserstreckung, und zwar außerhalb des vorderen oder hinteren Latzabschnitts gemeint. Die Erstreckung des Hüftgürtels in Längsrichtung ist vorzugsweise höchstens 25 cm. Besonders bevorzugt ist die Erstreckung im Bauchbereich geringer als im Rückenbereich, und der obere Rand des Hüftgürtels ist vorzugsweise leicht konturiert, um einen Bauchausschnitt zu bilden, der die Passform des Produkts verbessert sowie ein Rollen oder Umschlagen des Gürtels im Bauchbereich verhindert.

Hinsichtlich der Bemessung des Latzabschnitts erweist es sich als vorteilhaft, wenn der Latzabschnitt eine Erstreckung in Längsrichtung über eine distale Querkante des Bauch- oder Rückenbereichs in distaler Richtung auf den Schrittbereich von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 - 18 cm, insbesondere von 10 - 16 cm aufweist, wobei der vordere und hintere Latzabschnitt die gleiche oder eine unterschiedliche Erstreckung haben können. Die Erstreckung des Hüftgürtels in Längsrichtung beträgt im Bereich des Latzabschnitts vorne bei konturiertem Bauchausschnitt vorzugsweise 13 cm bis 19 cm, vorzugsweise 15 cm bis 17 cm, insbesondere 16 cm, im Bereich des Latzabschnitts hinten vorzugsweise 20 cm bis 27 cm, vorzugsweise 22 cm bis 25 cm, insbesondere 23,5 cm. Bei konturiertem Bauchausschnitt wurde dieser Wert ausgehend vom distalsten (tiefsten) Punkt der proximalen Querkante des Bauchbereichs bestimmt.

Die Erstreckung des Hüftgürtels in Querrichtung, das heisst, in Hüftumfangsrichtung beträgt zwischen 75 und 175 cm , vorzugsweise für die Größe S zwischen 50 und 90 cm, für die Größe M zwischen 80 und 120 cm, für die Größe L zwischen 100 und 140 cm, für die Größe XL zwischen 120 und 160 cm sowie für die Größe XXL zwischen 140 und 190 cm und orientiert sich an den Kleiderkonfektionsgrößen.

Nach einer bevorzugten Ausführungsform der Erfindung ist der betreffende Latzabschnitt einstückig mit dem Bauchbereich und/oder mit dem Rückenbereich des Hüftgürtels ausgebildet. Separat angestückte Latzabschnitte aus dem gleichen Material wie der übrige Hüftgürtel oder aus einem anderen Material als der übrige Hüftgürtel sind aber ebenfalls denkbar.

Der Bauchbereich und/oder Rückenbereich des Hüftgürtels ist vorzugsweise von einem Vliesmaterial oder Vliesverbundmaterial, insbesondere von einem im Wesentlichen undehnbaren Vliesmaterial oder Vliesverbundmaterial, gebildet.

Weiter erweist es sich als besonders vorteilhaft, wenn ein elastisch dehnbarer Seitenbereich des Hüftgürtels mit einem im wesentlichen undehnbaren Bauchbereich und einem im Wesentlichen undehnbaren Rückenbereich unlösbar verbunden ist. Vorzugsweise umfasst jeder Seitenbereich einen elastisch dehnbaren Abschnitt. In einer alternativen Ausführungsform kann es sich als vorteilhaft erweisen, wenn Teile des Rückenbereichs, insbesondere die seitlichen Teile des Rückenbereichs, die an die Seitenbereiche angefügt sind, elastische ausgebildet sind.

Darüber hinaus erweist es sich als vorteilhaft, wenn der vordere und/oder hintere Latzabschnitt farbig ausgebildet ist, wobei die Abschnitte vorzugsweise unterschiedlich farbig sind, um als visuelle Anlegehilfe zu dienen.

Weiterhin ist es vorteilhaft, wenn auf dem vorderen und/oder hinteren Latzabschnitt eine Markierung angebracht ist, die als Positionierhilfe für die Inkontinenzvorlage dient, derart, dass bei korrekt angelegter Inkontinenzvorlage und Fixier- und Tragevorrichtung die Markierung auf dem vorderen und/oder hinteren Latzabschnitt mit einer auf der äußeren Lage der Inkontinenzvorlage sichtbaren Markierung in Einklang gebracht ist. Beispielsweise kann eine Ausnehmung mittig am schrittzugewandten Rand des vorderen und/oder hinteren Latzabschnitts in Einklang gebracht werden mit einer Markierung, z.B. in Form eines in Längsrichtung mittig aufgebrachten Nässeindikators auf der äußeren Lage der Inkontinenzvorlage. Alternativ kann die Breite des unteren Rand des vorderen und/oder hinteren Latzabschnitts herstellerseitig so gewählt werden, dass diese mit dem Abstand von Markierungen auf der äußeren Lage der Inkontinenzvorlage, wie beispielsweise einer Größen- oder Saugstärken-Markierung, in Einklang gebracht werden kann.

Darüber hinaus betrifft die Erfindung ein System aus einer Fixier- und Tragevorrichtung nach der vorstehend beschriebenen Art mit einer daran lösbar festlegbaren Inkontinenzvorlage mit einer Längsrichtung und einer Querrichtung sowie zwei Längskanten und zwei Querkanten umfassend zwei auf der dem Benutzer zugewandten Seite der Inkontinenzvorlage in Längsrichtung der Inkontinenzvorlage verlaufenden Materialabschnitte, die entlang ihrer einen Längskante als Fixierungslinien an der Inkontinenzvorlage fixiert sind und deren zweite Längskante frei ist, wobei die Inkontinenzvorlage insbesondere zwei Längs- und zwei Querkanten umfasst und an der Fixier- und Tragevorrichtung so festgelegt ist, dass die Querkanten im Wesentlichen einer Querkante insbesondere der dem Schrittbereich abgewandten, also proximalen Querkante des Hüftgürtels entspricht.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Fixier- und Tragevorrichtung für eine wegwerfbare absorbierende Inkontinenzvorlage.

In der Zeichnung zeigt:
Figuren 1 und 2 eine Ausführungsform der erfindungsgemäßen Fixier- und Tragevorrichtung im angelegten Zustand zusammen mit einer wegwerfbaren absorbierenden Inkontinenzvorlage;
Figur 3 eine schematische Draufsicht auf die Fixier- und Tragevorrichtung im geöffneten und auf eine ebene Unterlage ausgebreiteten Zustand und
Figur 4 zeigt eine Fixier- und Tragevorrichtung in schematischer Darstellung mit daran einseitig festgelegter Inkontinenzvorlage
Figur 4a zeigt einen Schnitt der Linie A-A durch Figur 4
Figur 5 zeigt eine Fixier- und Tragevorrichtung mit einer hieran festgelegten alternativen Inkontinenzvorlage
Figur 5a zeigt einen Schnitt entlang der Linien A-A durch Figur 5
Figuren 6a,6b zeigen die Messvorrichtung zur Bestimmung der Biegesteifigkeit
Figuren 6c, 6d zeigen eine Aufsicht und einen Querschnitt des in die Messvorrichtung eingespannten Probenkörpers

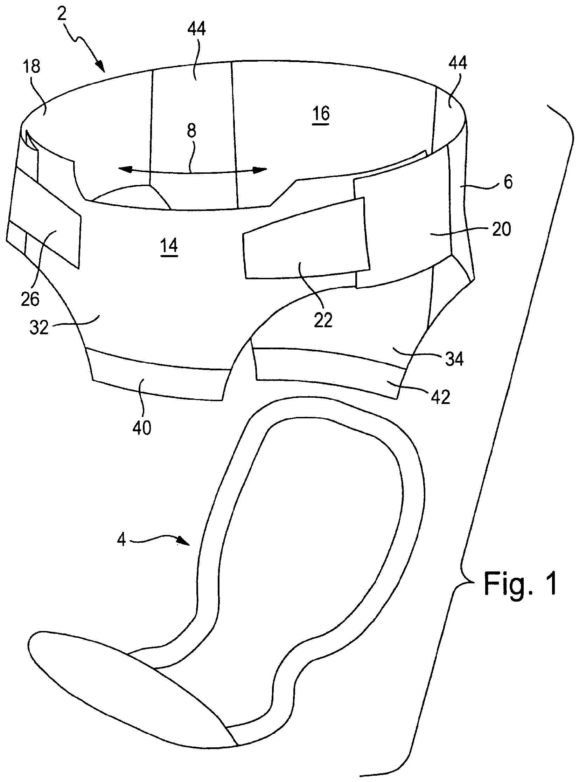

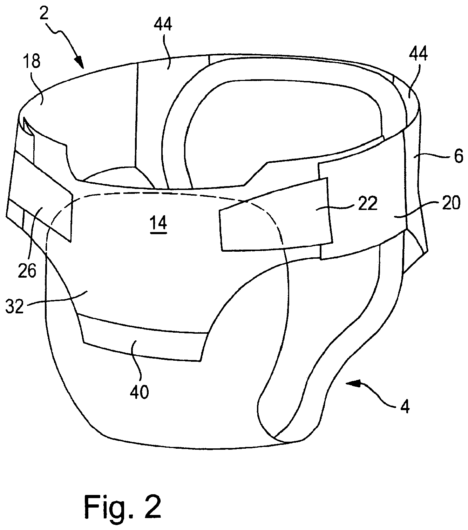

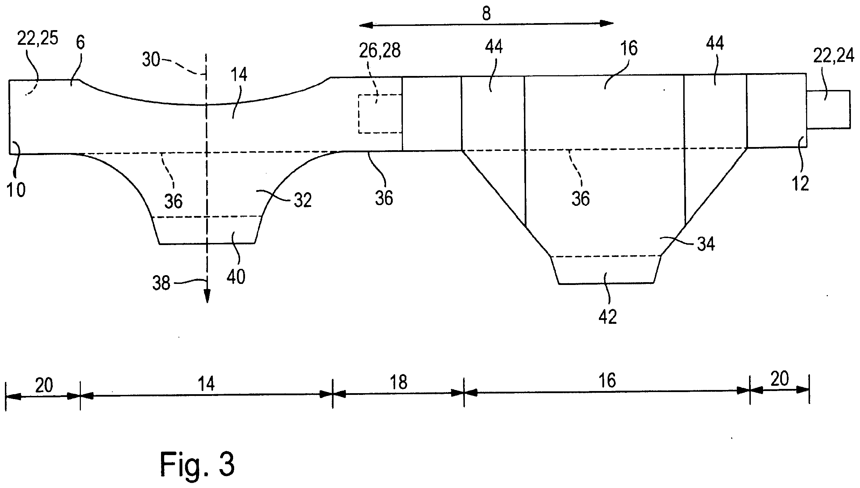

Die Figuren 1 und 2 zeigen perspektivische Ansichten einer insgesamt mit dem Bezugszeichen 2 bezeichneten Fixier- und Tragevorrichtung für eine mit Bezugszeichen 4 angedeutete absorbierende Inkontinenzvorlage, und zwar im an einen Benutzer angelegten Zustand. Die Fixier- und Tragevorrichtung 2 umfasst einen auf sich selbst lösbar schließbaren Hüftgürtel 6, der sich entlang einer Hüftumfangsrichtung 8 erstreckt und zwei freie Endabschnitte 10, 12 (s. Figur 3) aufweist, die zur Bildung der geschlossenen Hüftumfangsform auf sich selbst schließbar sind.

Die Fixier- und Tragevorrichtung 2 bzw. ihr Hüftgürtel 6 umfassen einen vorderen Bauchbereich 14, einen hinteren Rückenbereich 16 sowie einen linken Seitenbereich 18 und einen rechten Seitenbereich 20.

Zum Schließen des Hüftgürtels 6 sind an dessen freien Endabschnitten 10, 12 Gürtelverschlusselemente 22 vorgesehen, die im bevorzugt und beispielhaft dargestellten Fall von mechanisch wirkenden Verschlusselementen in Form eines Haken/Schlaufenverschlusssystems gebildet sind. Dabei ist in den Figuren mit Bezugszeichen 24 die hakenförmige Verschlusskomponente dargestellt, die über den Seitenrand 12 des Hüftgürtels hinausragt und mit einer schlaufenbildenden Komponente 25 in Form der äußeren Oberfläche des Bauchbereichs 14 lösbar haftend zusammenwirkt. Beispielsweise weist die Außenseite des Bauchbereichs 14 ein Vliesmaterial auf, welches die schlaufenförmige Komponente 25 des Verschlusssystems bildet.

Des Weiteren weist der nur an seinen Endabschnitten 10, 12, also nur an einer Stelle öffenbare Hüftgürtel 6 Sekundärverschlusselemente 26 auf, mittels derer die Umfangslänge des Hüftgürtels 6 einstellbar ist. Die Sekundärverschlusselemente 26 sind seitlich am Bauchbereich 14 bzw. am linken Seitenbereich 18 so vorgesehen, dass sie im angelegten Zustand der Fixier- und Tragevorrichtung (s. Figur 1) denselben optischen Eindruck wie die Gürtelverschlusselemente 22 hinsichtlich Ausbildung und Anordnung vermitteln, obschon ihre Funktion verschieden ist. Mittels der Sekundärverschlusselemente 26 werden nämlich keine öffenbaren und voneinander trennbaren Endabschnitte des Hüftgürtels 6 miteinander verbunden, sondern die Sekundärverschlusselemente 26 dienen lediglich dazu, den bestimmungsgemäßen Sitz des Hüftgürtels 6 an die Tragesituation anzupassen. Hierfür umfassen die Sekundärverschlusselemente 26 einen auf die Außenseite des Hüftgürtels 6 aufgebrachten Materialabschnitt 28, auf dem oder an dem sie selbst vorgesehen sind. Bei den Sekundärverschlusselementen 26 handelt es sich wiederum in bevorzugter Weise um mechanisch wirkende Verschlusselemente, insbesondere um Haken/Schlaufenmaterialien. Im beispielhaft dargestellten und bevorzugten Fall sind die Sekundärverschlusselemente 26 derart ausgebildet, dass an dem Materialabschnitt 28 eine hakenbildende Komponente des Verschlusssystems vorgesehen ist, die mit einer schlaufenbildenden Außenseite des Bauchbereichs 14 zusammenwirkt, so wie dies vorausgehend bei den Gürtelverschlusselementen 22 beschrieben wurde.

Als weitere wesentliche Komponente umfasst der Hüftgürtel 6 ausgehend vom Bauchbereich 14 und vom Rückenbereich 16 je einen symmetrisch zu einer Längsmittelachse 30 ausgebildeten vorderen und hinteren Latzabschnitt 32, 34. Die Latzabschnitte 32, 34 sind im beispielhaft bevorzugt dargestellten Fall einstückig mit dem Hüftgürtel 6 ausgebildet; sie erstrecken sich von einer schrittzugewandten oder unteren distalen Querkante 36 des Hüftgürtels entlang der Längsmittelachse 30 in Richtung 38 auf den Schrittbereich. Sie können sich in Richtung auf den Schrittbereich verjüngen und seitlich bogenförmig konturiert ausgebildet sein. An ihrem unteren, schrittzugewandten Endbereich umfassen die Latzabschnitte 32, 34 mechanisch wirkende Verschlusselemente 40, 42, und zwar auf der körperzugewandten Seite. Diese mechanisch wirkenden Verschlusselemente 40, 42 wirken lösbar haftend mit komplementären mechanisch wirkenden Verschlusselementen auf der körperabgewandten Seite der Inkontinenzvorlage 4 zusammen, um die Inkontinenzvorlage 4 lösbar, jedoch verliersicher an der Fixier- und Tragevorrichtung 2 festzulegen. Bevorzugtermaßen handelt es sich bei den Verschlusselementen 40, 42 um die hakenbildende Komponente eines mechanisch wirkenden Verschlusssystems, die mit der schlaufenbildenden Komponente auf der körperabgewandten Seite der Inkontinenzvorlage 4 zusammenwirkt. Die schlaufenbildende Komponente kann dabei in vorteilhafter Weise von einer Vliesbeschichtung, vorzugsweise der Vlieslage eines Vlies-Folien-Laminates, der Inkontinenzvorlage 4 gebildet sein.

Der Hüftgürtel 6 ist vorzugsweise aus Vlies- oder Vliesverbundmaterialien hergestellt. Im beispielhaft dargestellten jedoch bevorzugten Fall sind der Bauchbereich 14 und die beiden Seitenbereiche 18, 20 aus einem im Wesentlichen undehnbaren Vliesmaterial oder Vliesverbundmaterial gebildet, dessen Außenseite als schlaufenbildende Komponente für die Gürtelverschlusselemente 22 bzw. die Sekundärverschlusselemente 26 dient. Der Rückenbereich 16 ist zumindest teilweise elastisch dehnbar ausgebildet, indem er ein Paar elastisch dehnbare Materialabschnitte 44 aufweist, die bestimmungsgemäß unlösbar mit dem undehnbaren Material der Seitenbereiche 18, 20 verbunden sind und zwischen sich einen elastisch undehnbaren Bereich des Rückenbereichs einschließen.

Das Anlegen der Fixier- und Tragevorrichtung 2 zusammen mit der absorbierenden Inkontinenzvorlage 4 geschieht folgendermaßen: Zunächst wird der Hüftgürtel 6 bei bettlägerigen pflegebedürftigen Patienten durch eine Pflegeperson unter dem Körper des Patienten auf Hüfthöhe hindurchgeführt. Der Hüftgürtel 6 wird dabei so positioniert, dass der Rückenbereich 16 unterhalb des Gesäßes und der Bauchbereich 14 ungefähr vorne mittig zu liegen kommen. Sodann werden die freien Endabschnitte 10, 12 des Hüftgürtels 6 übereinander positioniert und mittels der Gürtelverschlusselemente 22 aufeinander geschlossen. Durch anschließendes Positionieren und Schließen des Materialabschnitts 28 und der Sekundärverschlusselemente 26 wird die Länge und Spannung des Hüftgürtels 6 in Umfangsrichtung 8 optimiert, so dass ein gleichmäßiger Zug auf den Bauchbereich 14 links und rechts ausgeübt wird und der Bauchbereich 14 wie auch der Rückenbereich 16 vorzugsweise symmetrisch in Bezug auf den Schrittbereich des Patienten und möglichst faltenfrei zu liegen kommen. Hierbei ist auch auf ein angenehmes Traggefühl abzustellen. Sodann wird der vordere und hintere Latzabschnitt 32, 34 mit der zuvor oder erst jetzt im Schrittbereich des Patienten positionierten Inkontinenzvorlage 4 unter Verwendung der mechanisch wirkenden Verschlusselemente 40, 42 lösbar haftend verbunden. Auch hierbei ist darauf zu achten, dass die vorderen und hinteren Latzabschnitte 32, 34 unter Ausübung einer gleichmäßigen und moderaten Zug- bzw. Haltekraft auf die Inkontinenzvorlage 4 fixiert werden, damit die Inkontinenzvorlage 4 in ihrer bestimmungsgemäßen symmetrischen Anordnung im Schrittbereich des Patienten verbleibt.

Hinsichtlich der Reihenfolge der vorausgehend geschilderten Schritte zum Anlegen des Hygieneartikels bzw. Inkontinenzsystems ist die Pflegeperson verhältnismäßig frei. Beispielsweise kann es sich in bestimmten Pflegesituationen als vorteilhaft erweisen, dass zunächst die Inkontinenzvorlage im Schrittbereich des Patienten vorpositioniert wird und erst dann der Hüftgürtel 6 auf sich selbst geschlossen wird. Es kann sich auch als zweckmäßig erweisen, dass die Sekundärverschlusselemente 26 erst nach dem Verbinden der Latzabschnitte 32, 34 mit der Inkontinenzvorlage 4 genutzt werden, um die optimale Spannung in Hüftumfangsrichtung 8 vorzugeben.

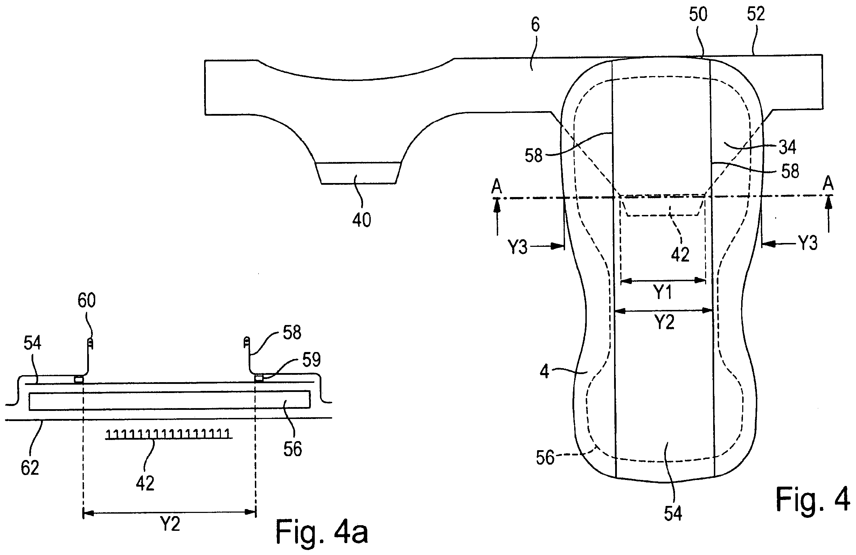

In Figur 4 ist nun eine Inkontinenzvorlage 4 gezeigt, die an dem Hüftgürtel 6 lediglich im Bereich des Latzabschnitts 34 des hinteren Rückenbereichs 16 festgelegt ist und dort mit dem Verschlusselement 42, das als mechanisches und/oder klebendes Verschlusselement ausgebildet ist und insbesondere ein Hakenelement eines Haken- und Schlaufenverschlusselementes umfasst über mechanisch und/oder klebend wirkende Verschlusselemente verbunden ist. Dabei sind die Verschlusselemente der Inkontinenzvorlage 4 durch das Material einer Rückenschicht (Backsheet) gebildet, das vorzugsweise ein Vliesmaterial umfasst, das nach außen gerichtet ist. Die Inkontinenzvorlage 4 wird dabei in Längsrichtung 38 so positioniert, dass eine ihrer Querkanten 50 mit einer proximalen Querkante 52 des Hüftgürtels 6 im Wesentlichen zusammenfällt. Die Inkontinenzvorlage 4 umfasst dabei eine Längs- und eine Querrichtung, die in der Zeichnungsebene mit der Längs- und der Querrichtung des Hüftgürtels 6 identisch ist. Dabei ist für die Längsrichtung auf die Längsrichtung des stehenden Trägers sowie auf die Längserstreckung der Inkontinenzvorlage 4 abzustellen.

Die Inkontinenzvorlage 4 umfasst dabei eine Rückenschicht sowie eine körperzugewandte Schicht 54 (Topsheet), die vorliegend gezeigt ist. Zwischen der Rückenschicht und der körperzugewandten Schicht 54 ist ein absorbierender Kern 56 eingeschlossen, der gestrichelt dargestellt ist. Darüber hinaus umfasst die Inkontinenzvorlage 4 zwei Materialabschnitte 58, deren Fixierungslinien 59 in Längsrichtung verlaufend zwischen sich den Abstand Y2 besitzen. Bei den Materialabschnitten 58 handelt es sich um im Wesentlichen rechteckige Materialbahnen, deren eine Längskante mit der köperzugewandten Schicht 54 als Fixierungslinie verbunden ist und deren andere Längskante zumindest über einen Teil der Länge frei insbesondere von der körperzugewandten Schicht 54 aufsteht, wobei hierzu eine Elastifizierung 60 vorgesehen sein kann. Es handelt sich hierbei um sogenannte Cuffs, die ein Austreten von Flüssigkeit aus der Inkontinenzvorlage 4 verhindern sollen insbesondere bei einem hohen Flüssigkeitsanfall auf der körperzugewandten Schicht 54 bis sämtliche Flüssigkeit durch die körperzugewandte Schicht 54 in den Saugkörper 56 aufgenommen ist.

Um zu verhindern, dass es bei einer Inkontinenzvorlage 4 zu einem Hineinziehen der Inkontinenzvorlage 4 in den Schrittbereich und somit zu einer Verkleinerung der für die Absorption zur Verfügung stehenden Fläche der körperzugewandten Schicht 54 durch ein Aufliegen der Materialabschnitte 58 auf der körperzugewandten Schicht 54 kommt, ist vorgesehen, dass die Verschlusselemente 42 und 40 zur Stabilisierung der Inkontinenzvorlage 4 dienen und so diese Aufspannen, so dass es nicht oder in einem geringeren Maße zu dem unerwünschten Effekt kommt.

Die Verschlusselemente 40, 42 dienen dabei zur Versteifung der Rückenschicht der Inkontinenzvorlage 4. Dazu ist es vorgesehen, dass die Breite der Verschlusselemente 40, 42 in Querrichtung, wobei die Verschlussemelemte insbesondere rechteckig oder trapezförmig ausgebildet sein können, wobei ihre Breite mit Y1 bezeichnet ist mindestens 50%, vorzugsweise jedoch mindestens 80% des Abstandes Y2 betragen. Dabei bezieht sich die Breite Y1 bei einem trapezförmigen Verschlusselement 40, 42 auf die lange Seite des Trapezes in Hüftumfangrichtung 8.

Auf diese Weise kann verhindert werden, dass es zu einem Zusammenfallen und Hineinziehen der Inkontinenzvorlage 4 in den Schrittbereich des Benutzers kommt. Um auf der anderen Seite zu erreichen, dass der Abstand der Verschlusselemente vom Rand der Inkontinenzvorlage, der hier mit Y3 bezeichnet ist, ausreichend ist, so dass es nicht zu einer Beeinträchtigung des Tragegefühls im Beinbereich durch die Verschlusselemente kommt, ist vorgesehen, dass die Breite der Verschlusselemente 40, 42 höchstens 80% von Y3 betragen soll. Ein weiterer Vorteil besteht darin, dass das Verschlusselement einen gleichmäßigen Zug auf den Schrittbereich der Inkontinenzvorlage ausübt und dieser damit über die gesamte Fläche zwischen den Fixierungslinien 59 der Materialabschnitte gleichmäßig in Richtung des Schrittbereiches des Benutzers gezogen wird und somit ein zu enges Hineinziehen der Inkontinenzvorlage in den Schrittbereich des Benutzers in der Mehrzahl der Tragesituationen verhindert werden kann.

Figur 4a zeigt einen Schnitt entlang der Linie A-A durch Figur 4, wobei hier gut das Aufstehen der Materialabschnitte 58 von der körperzugewandten Schicht 54 ersehen werden kann. Darüber hinaus kann auch die Elastifizierung 60 der sogenannten Cuffs erkannt werden.

Darüber hinaus zeigt Figur 4a auch die sogenannte Rückenschicht 62, die als Vliesmaterial ausgebildet ist und auf der das Verschlusselement 42 festgelegt ist.

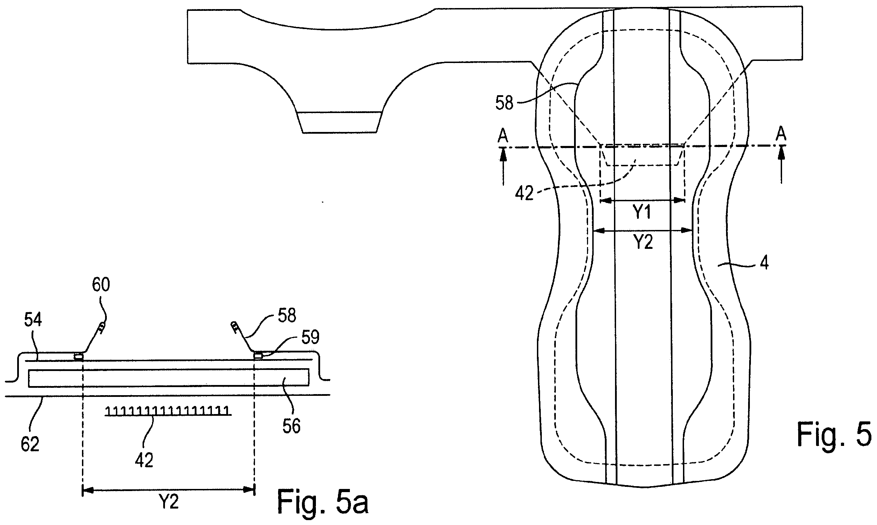

Figur 5 zeigt nun eine analoge Ausbildung jedoch hier mit einer sogenannten konturierten Fixierung der Materialabschnitte 58, wobei es hier zu einer Verjüngung und damit durch Verkleinerung des Abstandes Y2 im Schrittbereich sowie im vorderen und hinteren Teil der Inkontinenzvorlage 4 kommt. In diesem Fall soll der Wert Y1 im Wesentlichen mindestens 50% des Wertes Y2 an der schmalsten Stelle im Schrittbereich betragen.

Figur 5a zeigt dann wiederum eine entsprechende Schnittdarstellung gemäß Figur 5.

### Test zur Ermittlung der Biegesteifigkeit

Zur Ermittlung der Versteifung des Bereiches zwischen den Fixierungslinien der Materialabschnitte 58 der Inkontinenzvorlage 4 durch die Verschlusselementen 40, 42 der Latzabschnitte 32, 34 der Fixier- und Tragevorrichtung 2, die die Beabstandung der gegenüberliegenden Materialabschnitte 58 sicherstellt, wird die Biegesteifigkeit der Inkontinenzvorlage 4 selber und die durch das Verschlusselement 40, 42 versteifte Vorlage mit einem kommerziell erhältlichen Gerät zur Ermittlung der Biegesteifigkeit bestimmt.

### Probenvorbeitung

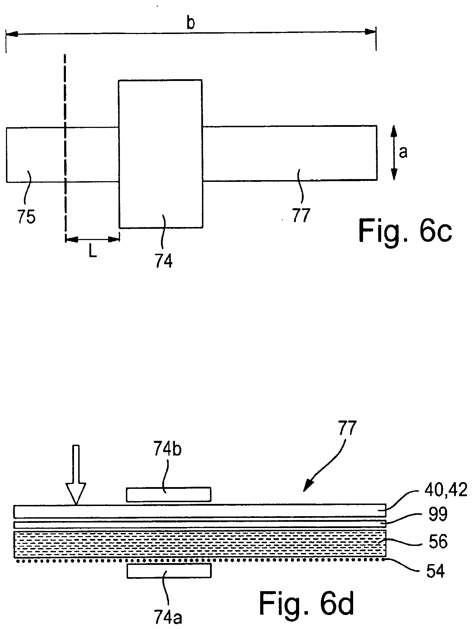

Die Fixier- und Tragevorrichtung wird bestimmungsgemäß, das heißt so, dass die proximale Kante des Bauch- und Rückenbereichs mit den Querkanten der Inkontinenzvorlage zusammenfällt, fixiert. Das Verschlusselement, bei dem es sich um ein Haken-Verschlusselement handelt, ist dann vorzugsweise auf dem an den Bauchbereich sich anschließenden Latzabschnitt 150 mm von der proximalen Kante entfernt und liegt zentriert auf der schlaufenbildenden Rückenschicht der Inkontinenzvorlage. Der Probenkörper 77 wird in einer Größe von a x b gleich 15 mm x 100 mm (Figur 6c) so aus dem Bereich der Verschlusselemente ausgestanzt, dass eine maximale Fläche des Verschlusselements ausgestanzt wird. Der Probenkörper wird mit einer 5 kg Rolle mit glatter Oberfläche viermal in 10 s angerollt. Für die Herstellung des Probenkörpers ohne Verschlusselement wird an der gleichen Stelle einer Vorlage eine Probe wie beschrieben genommen und angerollt.

### Probenvermessung

Für die Messungen wurde der Gerättyp 58963.013 der Firma Karl Frank GmbH, Weinheim-Birkenau, DE, verwendet. Es kann auch jedes ähnliche Gerät verwendet werden, wobei die Grundeinstellungen des Gerätes (Biegelänge (Kraftarm), Biegewinkel, Winkeldrehgeschwindigkeit) und auch der definierte Probenkörper beachtet werden müssen.

Die Messungen erfolgten mit einem Biegewinkel von 30° und einer Einpannlänge L (Abstand zwischen Biegeschneide und Spannklemme) von 15 mm. Die Einspanntiefe (Abschnittslänge des von der Spannklemme ausgehenden freien Probenkörpers) betrug 30 mm, so dass die Biegeschneide mittig ansetzt. Der Probenkörper wurde in der Spannklemme zentral eingespannt.

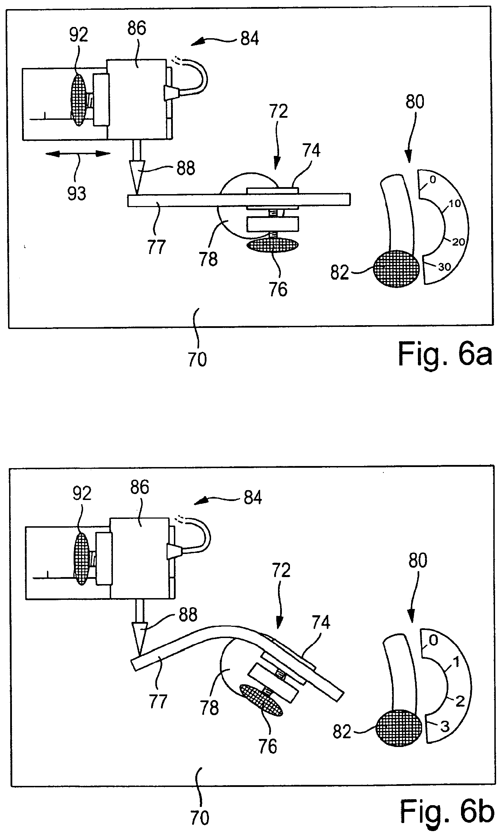

Das für die Biegesteifigkeitsmessung eingesetzte Gerät 70 ist in Figuren 6a und 6b schematisch dargestellt, inklusive eines Probekörpers 77 vor Beginn der Messung (Kraft gleich 0 mN, Figur 6a) und während der Messung (Kraft größer 0 mN, Figur 6b). Das Gerät 70 umfasst dabei einen Probenhalter 72 mit einer Einspannklemme 74 und einer Rändelschraube 76, die ein Zusammentreffen der beiden Einspannplatten 74a und 74b zur Befestigung des Probenkörpers 77 ermöglicht. Die Einspannklemme 74 ist dabei auf einer scheibenförmigen Platte 78 angebracht, wobei diese Platte 78 durch geräteinterne Funktionssteuerung bei der Durchführung der Messung eine Drehung im Uhrzeigersinn gemäß des eingegebenen Biegewinkels (hier 30°) durchführt. Die Drehwinkelgeschwindigkeit der Platte 78 beträgt 6 °/sec. Die Wahl des Biegewinkels kann dabei an einem weiteren Vorrichtungsbereich 80 festgelegt und mittels einer Rändelschraube 82 justiert werden. Die eigentliche Messvorrichtung 84 umfasst eine Messzelle 86. Hierin werden die durch einen Messfühler 88 aufgenommenen Kräfte in einen Kraftmesswert umgewandelt und letztlich als Messwert auf einem Display angezeigt. Der Messfühler 88 ist bei diesem Gerät in Form einer vertikal stehenden Schneide ausgeführt. Die schon erwähnte Biegelänge L (also die Länge des Kraftarms) ist dabei durch Justierung der Messvorrichtung 84 über eine Rändelschraube 92 in Richtung des Pfeils 93 einstellbar. Die Biegelänge L ist dabei zu verstehen als die Länge des Bereichs, der sich zwischen Messfühler und nächstliegender Kante der Einspannklemme 74 befindet und den Kraftarm bildet.

Vor Versuchsbeginn wird außerdem die Schneide des Messfühlers bis zur Berührung der Probe an die Probe herangeführt und justiert, so dass die Probe die Schneide des Messfühlers gerade berührt. Ein Überhang 75 des Probenkörpers 77 über die Schneide des Messfühlers beträgt ca. 15 mm.

Bei Durchführung der Messung dreht sich die Platte 78 mit der Einspannklemme 74 im Uhrzeigersinn bis zu dem angegebenen Biegewinkel, was dann zu einer Verformung des Probenkörpers führt, Figur 6b. Der Probenkörper 77 besteht aus einer Probe aus der Inkontinenzvorlage 4 ohne Verschlusselement und aus der Inkontinenzvorlage 4 mit einem Verschlusselement 40, 42 verstärkt, wobei für die Messungen Inkontinenzvorlagen der Marke MoliForm^{®} Premium Soft Super der Paul Hartmann Aktiengesellschaft, Heidenheim, Deutschland, Artikel-Nr. 168 919, verwendet wurden. Ein Probenkörper 77 aus mehrlagiger Vorlage aus körperzugewandter Schicht 54, absorbierendem Kern 56 und Rückenschicht 99 und Verschlusselementen 40, 42 ist in Figur 6d dargestellt in zwischen den Klemmbacken 74a, 74b eingespannter Konfiguration und eingerichteter Messzelle 88. Der Probenkörper wird mit dem Verschlusselement 40, 42 bzw. mit der Rückenschicht 99 der Inkontinenzvorlage 4 nach oben in Richtung der Messzelle eingespannt.

Der Probenkörper 77 wird gegen die Messzelle gebogen. Die durch die Verformung verursachten Kräfte werden in ablesbare Messdaten umgewandelt und an dem Display angezeigt.

Das Ergebnis einer Messung an jeweils 10 Probenkörpern ist in Tabelle 1 dargestellt.

### Tabelle 1 Biegesteifigkeiten [mN]

**Tabelle 1**

| Messung Nr. | B1 | B2 | |
|---|---|---|---|
| | Inkontinenzvorlage ohne Verschlusselement | Inkontinenzvorlage verstärkt mit Verschlusselement | |
| | mN | mN | |
| 1 | 249 | 2020 | |
| 2 | 287 | 2188 | |
| 3 | 324 | 2195 | |
| 4 | 330 | 2249 | |
| 5 | 354 | 2335 | |
| 6 | 427 | 2500 | |
| 7 | 462 | 2853 | |
| 8 | 464 | 3057 | |
| 9 | 512 | 3070 | |
| 10 | 672 | 3200 | B2:B1 |
| MITTELWERT | 408 | 2567 | 6,3 |
| MEDIAN | 391 | 2418 | 6,2 |

Die Kraft, die benötigt wird, um eine Inkontinenzvorlage mit auf der Rückenschicht fixiertem Verschlusselement 40,42 zu verformen, liegt im Durchschnitt bei 2.500 mN und ist damit viel größer, als bei der gleichen Vorlage ohne Verschlusselement, bei der die Biegesteifigkeit im Mittel nur bei 400 mN liegt. Somit liegt die Biegesteifigkeit der Inkontinenzvorlage mit Verschlusselement um etwa den Faktor 6 über der Biegesteifigkeit der Inkontinenzvorlage ohne Verschlusselement. Dies bedeutet, dass die Anordnung der Verschlusselemente auf der Inkontinenzvorlage zu einer höheren Rückstellkraft und Eigenstabilität führt und dadurch die Materialabschnitte auf der dem Benutzer zugewandten Seite der Inkontinenzvorlage sicher aufgespannt werden.

Die Streuung der Messwerte in beiden Messreihen ist vermutlich bedingt durch eine inhomogene Verteilung des superabsorbierenden Polymers (SAP) in der Inkontinenzvorlage, da sogenannte SAP-Nester im beladenen Zustand ebenfalls zu einer Versteifung führen können.

## Patentansprüche

1. System aus einer Fixier- und Tragevorrichtung (2) mit wegwerfbarer absorbierender Inkontinenzvorlage (4) mit einem mittels Gürtelverschlusselementen (22) auf sich selbst lösbar schließbaren und so eine in Hüftumfangsrichtung (8) durchgehende Hüftöffnung bildenden Hüftgürtel (6), an den die Inkontinenzvorlage (4) lösbar anbringbar ist, so dass sie im Schrittbereich des Benutzers getragen und nach Gebrauch wieder vom Hüftgürtel (6) entfernt und verworfen werden kann, wobei der Hüftgürtel (6) einen vorderen Bauchbereich (14), einen hinteren Rückenbereich (16) und einen linken und rechten Seitenbereich (18, 20) umfasst, der Hüftgürtel (6) ausgehend von dem Rückenbereich (16) und dem Bauchbereich (14) je einen sich in einer Längsrichtung in Richtung (38) auf den Schrittbereich des Benutzers erstreckenden Latzabschnitt (32, 34) aufweist, der an seiner körperzugewandten Seite Verschlusselemente (40, 42) aufweist, die mit komplementären Verschlusselementen auf der körperabgewandten Seite der Inkontinenzvorlage (4) lösbar haftend zusammenwirken, wobei die Inkontinenzvorlage (4) eine Längsrichtung und eine Querrichtung sowie zwei Längskanten und zwei Querkanten aufweist und an ihrer dem Benutzer zugewandten Seite ein Paar in Längsrichtung der Inkontinenzvorlage (4) verlaufende Materialabschnitte (58) aufweist, die entlang ihrer einen Längskante als Fixierungslinie (59) an der Inkontinenzvorlage (4) fixiert sind und deren zweite Längskante zumindest abschnittsweise frei ist, **dadurch gekennzeichnet**, dassdie Breite der Verschlusselemente (40, 42) quer zur Längsrichtung 60 bis 90% des Abstandes der Fixierungslinien (59) der Materialabschnitte (58) der Inkontinenzvorlage (4) beträgt, und wobei sich die Latzabschnitte (32, 34) in Richtung auf den Schrittbereich verjüngen.

2. Fixier- und Tragevorrichtung nach Anspruch 1 , **dadurch gekennzeichnet, dass** die Breite der Verschlusselemente (40, 42) quer zur Längsrichtung 70 bis 80%, weiter bevorzugt 74 bis 76 % des Abstandes der Fixierungslinien der Materialabschnitte (58) der Inkontinenzvorlage (4) beträgt.

3. Fixier- und Tragevorrichtung nach Anspruch 1 oder 2, **dadurch gekennzeichnet, dass** die Breite der Verschlusselemente (40, 42) quer zur Längsrichtung mindestens 30% und höchstens 90%, vorzugsweise mindestens 50% und höchstens 80 %, der Breite der Inkontinenzvorlage (4) im dem Bereich, in dem die Verschlusselemente (40, 42) mit der Inkontinenzvorlage (4) zusammenwirken, aufweisen.

4. Fixier- und Tragevorrichtung nach einem oder mehreren der vorangehenden Ansprüche, **dadurch gekennzeichnet, dass** die Inkontinenzvorlage (4) zwei Längs- und zwei Querkanten umfasst und im an der Fixier- und Tragevorrichtung (2) festgelegten Zustand, die Querkanten der Inkontinenzvorlage (4) im wesentlichen mit einer Querkante, insbesondere der dem Schrittbereich abgewandten, also proximalen Querkante des Hüftgürtels (6) zusammenfallen.

5. Fixier- und Tragevorrichtung nach einem oder mehreren der vorangehenden Ansprüche, **dadurch gekennzeichnet, dass** die Inkontinenzvorlage (4) hinsichtlich der Fixierungslinien so konturiert ist, dass die Fixierungslinien im Schrittbereich einen geringeren Abstand aufweisen als in einem Bereich außerhalb des Schrittbereichs.

6. Fixier- und Tragevorrichtung nach einem oder mehreren der vorangehenden Ansprüche, **dadurch gekennzeichnet, dass** die Verschlusselemente (40, 42) viereckig, insbesondere trapezförmig, insbesondere sich auf den Schrittbereich verjüngend ausgebildet sind.

7. Fixier- und Tragevorrichtung nach einem oder mehreren der vorangehenden Ansprüche, **dadurch gekennzeichnet, dass** die Verschlusselemente (40, 42) des Latzabschnitts (32, 34) auf einem streifenförmigen und quer zur Längsrichtung erstreckten Materialabschnitt vorgesehen sind, der an den Latzabschnitt (32, 34) angefügt ist.

8. Fixier- und Tragevorrichtung nach einem oder mehreren der vorangehenden Ansprüche, **dadurch gekennzeichnet, dass** die Verschlusselemente (40, 42) des Latzabschnitts (32, 34) vor der Ingebrauchnahme auf die körperzugewandte Seite des Latzabschnitts (32, 34) eingeschlagen sind und vorzugsweise dort haftend fixiert sind.

9. Fixier- und Tragevorrichtung nach einem oder mehreren der vorangehenden Ansprüche, **dadurch gekennzeichnet, dass** der Hüftgürtel (6) nur in einem Bereich öffen- und schließbar und die Gürtelverschlusselemente (22, 24) in diesem Bereich an freien Endabschnitten des Hüftgürtels (6) vorgesehen sind.

10. Fixier- und Tragevorrichtung nach einem oder mehreren der vorangehenden Ansprüche, **dadurch gekennzeichnet, dass** Sekundärverschlusselemente (26) vorgesehen sind, mittels derer eine Umfangslänge des Hüftgürtels (6) einstellbar und so zumindest der vordere Latzabschnitt (32, 34) symmetrisch zum Schritt des Benutzers positionierbar ist, wobei die Sekundärverschlusselemente (26) beidseits des Bauchbereichs (14) oder im Seitenbereich des Hüftgürtels (6), gegenüberliegend den Gürtelverschlusselementen (22) vorgesehen sind.

11. Fixier- und Tragevorrichtung nach einem oder mehreren der vorangehenden Ansprüche, **dadurch gekennzeichnet, dass** die Gürtelverschlusselemente (22) und/oder die Sekundärverschlusselemente (26) und/oder die Verschlusselemente (40, 42) am vorderen und hinteren Latzabschnitt (32, 34) mechanische und/oder klebende Verschlusselemente sind.

12. System nach einem der vorangehenden Ansprüche, **dadurch gekennzeichnet, dass** die Inkontinenzvorlage (4) zwei Längs- und zwei Querkanten umfasst und im an der Fixier- und Tragevorrichtung festgelegten Zustand, die Querkanten im wesentlichen mit einer Querkante, insbesondere der dem Schrittbereich abgewandten, also proximalen Querkante des Hüftgürtels (6) zusammenfallen.

## Claims

1. System consisting of a fixing and supporting device (2) with a disposable, absorbent incontinence pad (4), comprising a hip belt (6) which is able to be releasably closed on itself by means of belt closure elements (22) and thus forms a hip opening which is continuous in the hip circumferential direction (8), to which belt the incontinence pad (4) can be releasably attached such that it can be worn in the crotch region of the user and removed again from the hip belt (6) after use, the hip belt (6) having a front abdominal region (14), a rear back region (16), and a left and right lateral region (18, 20), the hip belt (6) having, starting from the back region (16) and the abdominal region (14), a flap portion (32, 34) in each case which extends in a longitudinal direction in the direction (38) toward the crotch region of the user and has closure elements (40, 42) on its side facing the body which interact in a releasably adhering manner with complementary closure elements on the side of the incontinence pad (4) facing away from the body, the incontinence pad (4) having a longitudinal direction and a transverse direction, as well as two longitudinal edges and two transverse edges and, on its side facing the user, a pair of material portions (58) which extend in the longitudinal direction of the incontinence pad (4) and are fixed to the incontinence pad (4) along their one longitudinal edge as a fixing line (59) and the second longitudinal edge of which is at least partially free, **characterized in that** the width of the closure elements (40, 42) transversely to the longitudinal direction is 60 to 90% of the distance between the fixing lines (59) of the material portions (58) of the incontinence pad (4), and with the flap portions (32, 34) tapering in the direction of the crotch region.

2. Fixing and supporting device according to claim 1, **characterized in that** the width of the closure elements (40, 42) transversely to the longitudinal direction is 70 to 80%, more preferably 74 to 76% of the distance between the fixing lines of the material portions (58) of the incontinence pad (4).

3. Fixing and supporting device according to claim 1 or 2, **characterized in that** the width of the closure elements (40, 42) transversely to the longitudinal direction is at least 30% and at most 90%, preferably at least 50% and at most 80% of the width of the incontinence pad (4) in the region in which the closure elements (40, 42) interact with the incontinence pad (4).

4. Fixing and supporting device according to one or more of the preceding claims, **characterized in that** the incontinence pad (4) comprises two longitudinal and two transverse edges and, in the state secured to the fixing and supporting device (2), the transverse edges of the incontinence pad (4) coincide substantially with a transverse edge, in particular the transverse edge of the hip belt (6) facing away from the crotch region, i.e. the proximal transverse edge.

5. Fixing and supporting device according to one or more of the preceding claims, **characterized in that** the incontinence pad (4) is contoured with respect to the fixing lines in such a way that the fixing lines have a smaller distance in the crotch region than in a region outside the crotch region.

6. Fixing and supporting device according to one or more of the preceding claims, **characterized in that** the closure elements (40, 42) are square, in particular trapezoidal, in particular designed to taper to the crotch region.

7. Fixing and supporting device according to one or more of the preceding claims, **characterized in that** the closure elements (40, 42) of the flap portion (32, 34) are provided on a strip-shaped material portion which extends transversely to the longitudinal direction and is attached to the flap portion (32, 34).

8. Fixing and supporting device according to one or more of the preceding claims, **characterized in that** the closure elements (40, 42) of the flap portion (32, 34) are inserted into the side of the flap portion (32, 34) facing the body before use and are preferably adhesively fixed there.

9. Fixing and supporting device according to one or more of the preceding claims, **characterized in that** the hip belt (6) can be opened and closed only in one region, and the belt closure elements (22, 24) are provided in this region on free end portions of the hip belt (6).

10. Fixing and supporting device according to one or more of the preceding claims, **characterized in that** secondary closure elements (26) are provided by means of which a circumferential length of the hip belt (6) can be adjusted and thus at least the front flap portion (32, 34) can be positioned symmetrically with respect to the crotch of the user, the secondary closure elements (26) being provided on both sides of the abdominal region (14) or in the lateral region of the hip belt (6), opposite the belt closure elements (22) .

11. Fixing and supporting device according to one or more of the preceding claims, **characterized in that** the belt closure elements (22) and/or the secondary closure elements (26) and/or the closure elements (40, 42) on the front and rear flap portion (32, 34) are mechanical and/or adhesive closure elements.

12. System according to any of the preceding claims, **characterized in that** the incontinence pad (4) comprises two longitudinal and two transverse edges and, in the state secured to the fixing and supporting device, the transverse edges coincide substantially with a transverse edge, in particular the transverse edge of the hip belt (6) facing away from the crotch region, i.e. the proximal transverse edge.

## Revendications

1. Système composé d'un dispositif de fixation et de support (2) avec une protection pour incontinence (4) absorbante jetable avec une ceinture de hanches (6) pouvant être fermée sur elle-même de manière amovible au moyen d'éléments de fermeture de ceinture (22) et formant ainsi une ouverture continue au niveau des hanches dans la direction périphérique des hanches (8), sur laquelle la protection pour incontinence (4) peut être placée de manière amovible, de sorte qu'elle peut être portée dans la région de l'entrejambes de l'utilisateur et de nouveau enlevée de la ceinture de hanches (6) après utilisation et jetée, dans lequel la ceinture de hanches (6) comprend une région ventrale (14) avant, une région dorsale (16) arrière et une région latérale (18, 20) gauche et droite, la ceinture de hanches (6) présente à partir de la région dorsale (16) et de la région ventrale (14) respectivement une partie patte (32, 34) s'étendant dans une direction longitudinale en direction (38) de la région de l'entrejambes de l'utilisateur, qui présente sur sa face tournée vers le corps des éléments de fermeture (40, 42), qui coopèrent par adhérence de manière amovible avec des éléments de fermeture complémentaires sur la face de la protection pour incontinence (4) opposée au corps, dans lequel la protection pour incontinence (4) présente une direction longitudinale et une direction transversale ainsi que deux bords longitudinaux et deux bords transversaux et présente sur sa face tournée vers l'utilisateur une paire de parties de matériau (58) s'étendant dans la direction longitudinale de la protection pour incontinence (4), qui sont fixées le long de l'un de leurs bords longitudinaux en tant que ligne de fixation (59) sur la protection pour incontinence (4) et dont leur deuxième bord longitudinal est libre au moins sur certaines parties, **caractérisé en ce que** la largeur des éléments de fermeture (40, 42) transversalement par rapport à la direction longitudinale atteint 60 à 90 % de l'écart entre les lignes de fixation (59) des parties de matériau (58) et la protection pour incontinence (4), et dans lequel les parties pattes (32, 34) s'amincissent en direction de la région de l'entrejambes.

2. Dispositif de fixation et de support selon la revendication 1, **caractérisé en ce que** la largeur des éléments de fermeture (40, 42) transversalement par rapport à la direction longitudinale atteint 70 à 80 %, plus préférablement 74 à 76 % de l'écart entre les lignes de fixation des parties de matériau (58) et la protection pour incontinence (4).

3. Dispositif de fixation et de support selon la revendication 1 ou 2, **caractérisé en ce que** la largeur des éléments de fermeture (40, 42) transversalement par rapport à la direction longitudinale atteint au moins 30 % et au plus 90 %, de préférence au moins 50 % et au plus 80 %, de la largeur de la protection pour incontinence (4) dans la région dans laquelle les éléments de fermeture (40, 42) coopèrent avec la protection pour incontinence (4).

4. Dispositif de fixation et de support selon l'une ou plusieurs des revendications précédentes, **caractérisé en ce que** la protection pour incontinence (4) comprend deux bords longitudinaux et deux bords transversaux et dans l'état fixé sur le dispositif de fixation et de support (2), les bords transversaux de la protection pour incontinence (4) coïncident sensiblement avec un bord transversal, en particulier le bord transversal de la ceinture de hanches (6) opposé à la région de l'entrejambes, par conséquent proximal.

5. Dispositif de fixation et de support selon l'une ou plusieurs des revendications précédentes, **caractérisé en ce que** la protection pour incontinence (4) présente un contour en ce qui concerne les lignes de fixation tel que les lignes de fixation dans la région de l'entrejambes présentent un écart plus petit que dans une région à l'extérieur de la région de l'entrejambes.

6. Dispositif de fixation et de support selon l'une ou plusieurs des revendications précédentes, **caractérisé en ce que** les éléments de fermeture (40, 42) sont réalisés de manière carrée, en particulier trapézoïdale, en particulier de manière à s'amincir sur la région de l'entrejambes.

7. Dispositif de fixation et de support selon l'une ou plusieurs des revendications précédentes, **caractérisé en ce que** les éléments de fermeture (40, 42) de la partie patte (32, 34) sont prévus sur une partie de matériau en forme de bande et étendue transversalement par rapport à la direction longitudinale, qui est jointe à la partie patte (32, 34).

8. Dispositif de fixation et de support selon l'une ou plusieurs des revendications précédentes, **caractérisé en ce que** les éléments de fermeture (40, 42) de la partie patte (32, 34) sont enfoncés avant l'utilisation sur la face de la partie patte (32, 34) tournée vers le corps et y sont de préférence fixés par adhérence.

9. Dispositif de fixation et de support selon l'une ou plusieurs des revendications précédentes, **caractérisé en ce que** la ceinture de hanches (6) ne peut être ouverte et fermée que dans une région et les éléments de fermeture de ceinture (22, 24) sont prévus dans cette région sur des parties d'extrémité libres de la ceinture de hanches (6).

10. Dispositif de fixation et de support selon l'une ou plusieurs des revendications précédentes, **caractérisé en ce que** des éléments de fermeture secondaires (26) sont prévus, au moyen desquels une longueur circonférentielle de la ceinture de hanches (6) est réglable et ainsi au moins la partie patte (32, 34) avant peut être positionnée de manière symétrique par rapport à l'entrejambe de l'utilisateur, dans lequel les éléments de fermeture secondaires (26) sont prévus de part et d'autre de la région ventrale (14) ou dans la région latérale de la ceinture de hanches (6), à l'opposé des éléments de fermeture de ceinture (22).

11. Dispositif de fixation et de support selon l'une ou plusieurs des revendications précédentes, **caractérisé en ce que** les éléments de fermeture de ceinture (22) et/ou les éléments de fermeture secondaires (26) et/ou les éléments de fermeture (40, 42) sur la partie patte (32, 34) avant et arrière sont des éléments de fermeture mécaniques et/ou adhésifs.

12. Système selon l'une quelconque des revendications précédentes, **caractérisé en ce que** la protection pour incontinence (4) comprend deux bords longitudinaux et deux bords transversaux et dans l'état fixé sur le dispositif de fixation et de support, les bords transversaux coïncident sensiblement avec un bord transversal, en particulier le bord transversal de la ceinture de hanches (6) opposé à la région de l'entrejambes, par conséquent proximal.
